# EUROPÄISCHE PATENTSCHRIFT

(11) **EP 3 311 791 B1**
(45) Veröffentlichungstag und Bekanntmachung des Hinweises auf die Patenterteilung: **17.06.2020**
(21) Anmeldenummer: 17194945.6
(22) Anmeldetag: 05.10.2017
(51) Int. Cl.: A61K 8/34, A61K 8/35, A61K 8/37, A61K 8/41, A61K 8/49, A61Q 17/04

(54) **ETHANOLISCHES SONNENSCHUTZMITTEL MIT REDUZIERTER NEIGUNG ZUR TEXTILVERFLECKUNG**
ETHANOL SUNSCREEN COMPOSITION WITH A REDUCED LIKELIHOOD OF DISCOLOURING TEXTILES
INHIBITEUR DE LUMIÈRE ÉTHANOLIQUE PRÉSENTANT UNE TENDANCE RÉDUITE À TÂCHER LES TISSUS

(30) Priorität: 20.10.2016 DE 102016220547
(43) Veröffentlichungstag der Anmeldung: 25.04.2018
(73) Patentinhaber: Beiersdorf AG, 20253 Hamburg (DE)
(72) Erfinder: Borchers, Kathrin, 21614 Buxtehude (DE); Toshihiko, Shimoda, Tokyo (JP); Eisert, Anja, 22085 Hamburg (DE); von der Fecht, Stephanie, 22880 Wedel (DE)

(56) Entgegenhaltungen:
- EP-A1- 2 939 710
- WO-A1-2011/101250
- DE-A1-102005 059 742
- DE-A1-102006 029 837
- US-A1- 2014 328 777
- "Highly efficient suncare and daily care compositions", IP.COM JOURNAL, IP.COM INC., WEST HENRIETTA, NY, US, 5 May 2015 (2015-05-05), XP013167261, ISSN: 1533-0001
- "Sunscreen", IP.COM JOURNAL, IP.COM INC., WEST HENRIETTA, NY, US, 4 February 2011 (2011-02-04), XP013142406, ISSN: 1533-0001
- Anonymous: "Suncare Compositions (2)", IP.com Prior Art Database Technical Disclosure, 10 July 2012 (2012-07-10), pages 1-74, XP055611163, Retrieved from the Internet: URL:https://priorart.ip.com/IPCOM/00021973 6 [retrieved on 2019-08-06]
- Anonymous: "Highly efficient suncare and daily care compositions (7)", IP.com Prior Art Database Technical Disclosure, 20 August 2015 (2015-08-20), pages 1-38, XP055611167, Retrieved from the Internet: URL:https://priorart.ip.com/IPCOM/00024281 3 [retrieved on 2019-08-06]

## Beschreibung

Die vorliegende Erfindung betrifft ein ethanolisches, kosmetisches Sonnenschutzmittel enthaltend
a) einen oder mehrere UV-Filter gewählt aus der Gruppe der Verbindungen 4-(tert.-Butyl)-4'-methoxydibenzoylmethan (INCI Butyl Methoxydibenzoylmethane), (2-[-4-(Diethylamino)-2-hydroxybenzoyl]Benzoesäurehexylester (INCI: Diethylamino Hydroxybenzoyl Hexyl Benzoate) und 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazine),
b) Ethanol in einer Mindestmenge von 10 Gewichts-%, bezogen auf das Gesamtgewicht der Zubereitung,
c) ein oder mehrere Ölkomponenten mit einem Kontaktwinkel von größer/gleich 44°,
sowie ein Verfahren und die Verwendung von einer oder mehrerer Ölkomponenten mit einem Kontaktwinkel von größer/gleich 44° zum Schutz vor Textilverfleckung.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurde daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Kosmetische Zubereitungen wie Sonnenschutzzubereitungen, die auf die Haut aufgetragen werden, kommen regelmäßig (beabsichtigt oder unbeabsichtigt) mit Kleidungsstücken und Wäschestücken (z.B. Handtücher) in Kontakt, an denen sie (z.B. als "Abrieb" oder weil sie von den Faserstoffen "aufgesaugt" werden) zum Teil haften bleiben. Auf diese Weise entstehen, je nach Art der Inhaltsstoffe, insbesondere auf hellen Textilien Flecken und Verfärbungen. Diese Verfärbungen werden insbesondere durch nicht-wasserlösliche UVA- und Breitbandfilter wie 4-(tert.-Butyl)-4'-methoxydibenzoylmethan (INCI Butyl Methoxydibenzoylmethane), (2-[-4-(Diethylamino)-2-hydroxybenzoyl] Benzoesäurehexylester (INCI: Diethylamino Hydroxybenzoyl Hexyl Benzoate) und 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin) hervorgerufen. Die Verfleckungen sind durch Waschen mit herkömmlichen Waschmitteln kaum zu entfernen und verstärken sich während des Waschprozesses durch Wechselwirkungen mit Ionen des Waschwassers sogar noch.

Zwar wurden in jüngerer Zeit eine Reihe von Lösungswegen entwickelt, dieses Textilverfleckungsproblem durch die Auswahl bestimmter Inhaltsstoffe für Sonnenschutzmittel in den Griff zu bekommen. Nachteilig an diesen Lösungen ist jedoch der Umstand, dass sie nur in Emulsionssystemen wirksam sind, bei Gelen und Sprays auf Ethanol-Basis jedoch versagen. Da diese meist transparenten Sonnenschutzmittel sich jedoch beim Verbraucher wachsender Beliebtheit erfreuen, war es die Aufgabe der vorliegenden Erfindung, ein solches ethanolisches Sonnenschutzmittel mit reduzierter Neigung zur Textilverfleckung zu entwickeln. Insbesondere war es die Aufgabe ein Sonnenschutzmittel auf Ethanol-Basis mit reduzierter Textilverfleckung zu entwickeln, welches einen oder mehrere UV-Filter gewählt aus der Gruppe der Verbindungen 4-(tert.-Butyl)-4'-methoxydibenzoylmethan (INCI Butyl Methoxydibenzoylmethane), (2-[-4-(Diethylamino)-2-hydroxybenzoyl]Benzoesäurehexylester (INCI: Diethylamino Hydroxybenzoyl Hexyl Benzoate) und 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin) enthält. Dabei sollte das Sonnenschutzmittel einen hohen Lichtschutzfaktor aufweisen, d.h. einen Lichtschutzfaktor von mindestens 20, bevorzugt von mindestens 30 und besonders bevorzugt von 50 aufweisen.

Gelöst wird die Aufgabe durch ein ethanolisches, kosmetisches Sonnenschutzmittel enthaltend
a) einen oder mehrere UV-Filter gewählt aus der Gruppe der Verbindungen 4-(tert.-Butyl)-4'-methoxydibenzoylmethan (INCI Butyl Methoxydibenzoylmethane), (2-[-4-(Diethylamino)-2-hydroxybenzoyl]Benzoesäurehexylester (INCI: Diethylamino Hydroxybenzoyl Hexyl Benzoate) und 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazine),
b) Ethanol in einem Gehalt von 20 bis 75 Gewichts-%, bezogen auf das Gesamtgewicht der Zubereitung,
c) ein oder mehrere Ölkomponenten mit einem Kontaktwinkel von größer/gleich 44°, wobei als Ölkomponenten mit einem Kontaktwinkel von größer/gleich 44° eine oder mehrere Verbindungen aus der Gruppe C12-15 Alkylbenzoate (INCI: C12-15 Alkyl Benzoate), 2-Octyldodecan-1-ol (INCI: Octyldodecanol), Capryl/Caprinsäure Triglyceride (INCI: Caprylic/Capric Triglyceride) gewählt werden und die Ölkomponenten mit einem Kontaktwinkel von größer/gleich 44° in einer Gesamtkonzentration von 15 bis 60 Gewichts-%, bezogen auf das Gesamtgewicht der Zubereitung,
d) 2-Ethylhexyl 2-hydroxybenzoat (INCI: Ethylhexyl Salicylate) und/oder 3,3,5-Trimethylcyclohexyl 2-hydroxybenzoat (INCI: Homosalate), wobei die Zubereitung kein 3-(4-Methylbenzyliden)-campher, 2-Hydroxy-4-methoxybenzophenon (INCI: Oxybenzon), Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze, 3-Benzylidencampher; Terephthalidendicampher-sulfonsäure; 4-(Dimethylamino)-benzoesäure(2-ethylhexyl)ester; 4-Methoxybenzalmalon-säuredi(2-ethylhexyl)ester; 4-Methoxyzimtsäureisoamylester und 4-Methoxyzimtsäure-ethylhexylester enthält, also frei von diesen Inhaltsstoffen ist.

Gelöst wird die Aufgabe ferner durch die Verwendung von Ölkomponenten mit einem Kontaktwinkel von größer/gleich 44°° aus der Gruppe C12-15 Alkylbenzoate (INCI: C12-15 Alkyl Benzoate), 2-Octyldodecan-1-ol (INCI: Octyldodecanol), Capryl/Caprinsäure Triglyceride (INCI: Caprylic/Capric Triglyceride) in ethanolischen, kosmetischen Sonnenschutzmitteln enthaltend einen oder mehrere UV-Filter aus der Gruppe der Verbindungen 4-(tert.-Butyl)-4'-methoxydibenzoylmethan (INCI Butyl Methoxydibenzoylmethane), (2-[-4-(Diethylamino)-2-hydroxybenzoyl]Benzoesäurehexylester (INCI: Diethylamino Hydroxybenzoyl Hexyl Benzoate) und 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazine), zur Erleichterung der Auswaschbarkeit der UV-Lichtschutzfilter aus mit dem Sonnenschutzmittel kontaminierten Textilien.

Gelöst wird die Aufgabe durch die Verwendung von Ölkomponenten mit einem Kontaktwinkel von größer/gleich 44°° aus der Gruppe C12-15 Alkylbenzoate (INCI: C12-15 Alkyl Benzoate), 2-Octyldodecan-1-ol (INCI: Octyldodecanol), Capryl/Caprinsäure Triglyceride (INCI: Caprylic/Capric Triglyceride) in ethanolischen, kosmetischen Sonnenschutzmitteln enthaltend einen oder mehrere UV-Filter aus der Gruppe der Verbindungen 4-(tert.-Butyl)-4'-methoxydibenzoylmethan (INCI Butyl Methoxydibenzoylmethane), (2-[-4-(Diethylamino)-2-hydroxybenzoyl]Benzoesäurehexylester (INCI: Diethylamino Hydroxybenzoyl Hexyl Benzoate) und 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazine), zur Reduzierung der durch das Sonnenschutzmittel ausgelösten Textilverfleckungen

Gelöst wird die Aufgabe nicht zuletzt durch ein Verfahren zur Erleichterung der Auswaschbarkeit von UV-Filter-Rückständen aus Textilien, die mit einem ethanolischen, kosmetischen Sonnenschutzmittel kontaminiert wurden, wobei dem ethanolischem Sonnenschutzmittel enthaltend einen oder mehrere UV-Filter aus der Gruppe der Verbindungen 4-(tert.-Butyl)-4'-methoxydibenzoylmethan (INCI Butyl Methoxydibenzoylmethane), (2-[-4-(Diethylamino)-2-hydroxybenzoyl]Benzoesäurehexylester (INCI: Diethylamino Hydroxybenzoyl Hexyl Benzoate) und 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazine), ein oder mehrere Ölkomponenten mit einem Kontaktwinkel von größer/gleich 44°° aus der Gruppe C12-15 Alkylbenzoate (INCI: C12-15 Alkyl Benzoate), 2-Octyldodecan-1-ol (INCI: Octyldodecanol), Capryl/Caprinsäure Triglyceride (INCI: Caprylic/Capric Triglyceride) zugesetzt wird.

Zwar kennt der Stand der Technik die EP 2 939 710 A1, DE 10 2005 059742 A1, WO 2011/101250 A1, DE 10 2006 029837 A1 und US 2014/328777 A1, sowie die IP.Com Veröffentlichungen IP.com number IPCOM000203831D, IPCOM000241483D, IPCOM000219736D und IPCOM000242813D doch konnten diese Schriften nicht den Weg zur vorliegenden Erfindung weisen.

Als "ethanolisches Sonnenschutzmittel" wird im Rahmen der vorliegenden Erfindung ein Sonnenschutzmittel verstanden, dass von 20 bis 75 Gewichts-% Ethanol, bezogen auf das Gesamtgewicht der Zubereitung, enthält. Erfindungsgemäß bevorzugt enthält ein solches ethanolisches Sonnenschutzmitlei von 25 bis 70 Gewichts-% Ethanol, jeweils bezogen auf das Gesamtgewicht der Zubereitung.

Die Formulierungen "erlindungsgernäß", "erfindungsgemäß vorteilhaft" etc. beziehen sich im Rahmen der vorliegenden Offenbarung immer auf das erfindungsgemäße Sonnenschutzmittel, die erfindungsgemäßen Verwendungen und das erfindungsgemäße Verfahren, wenn es im Einzelfall nicht anders beschrieben wird.

Die erfindungsgemäße Kontaktwinkel-Bestimmung erfolgt erfindungsgemäß nach dem folgenden Verfahren:
Messung des Kontaktwinkefs von Lipiden auf Glasobjektträgem.

Die Messungen erfolgen mit einem optischen Kontaktwinkelmessgerät Dataphysics OCA 20, Verwendet wird die Methode des liegenden Troptens (sessile drop). Bei diesem Verfahren wird ein Tropfen einer definierten Flüssigkeit von 10 µL auf den Glasobjektträger aufgebracht. Der Schattenriss des sich auf der Oberfläche ausbildenden Tropfens wird mit einer CCD-Kamera aufgenommen. An dem im Computer eingelesenen Bild wird eine Konturanalyse vorgenommen. Durch Anlegen einer Basislinie und einer Tangente bestimmt die Software automatisch den Kontaktwinkel des auf den Glasobjektträger liegenden Tropfens, Über einen Zeitraum von 3 s wird mit einer Framerate von 6,25 Bildern pro Sekunde der Kontaktwinkel ermittelt Ausgewertet wird jeweils der mittlere Kontaktwinkel. Zur Ermittlung des Messwertes werden je fünf Messungen durchgeführt und das Ergebnis statistisch aufbereitet.

**Tabelle 1: Kontaktwinkel einiger in der Kosmetik verwendeter Olkomponenten**

| INCI | Kontaktwinkel in ° |
|---|---|
| Cyclomethicone | 25,84 |
| Dibutyl Adipate | 34,72 |
| Butylene Glycol Caprylate/Caprate | 40,82 |
| C12-15 Alkyl Benzoate | 44,025 |
| Octyldodecanol | 52,20 |
| Caprylic/Capric Triglyceride | |

Enthält die erfindungsgemäße Zubereitung C12-15 Alkylbenzoate (INCI: C12-15 Alkyl Benzoate), so ist es vorteilhaft im Sinne der vorliegenden Erfindung, diese Substanz in einer Konzentration von 4 bis 15 Gewichts-%, bezogen auf das Gesamtgewicht der Zubereitung, einzusetzen.

Enthält die erfindungsgemäße Zubereitung 2-Octyldodecan-1-ol (INCI; Octyldodecanol, so ist es vorteilhaft im Sinne der vorliegenden Erfindung, diese Substanz in einer Konzentration von 6 bis 25 Gewichts-%, bezogen auf das Gesamtgewicht der Zubereitung, einzusetzen.

Enthält die erfindungsgemäße Zubereitung Capryl/Caprinsäure Triglyceride (INCI: Caprylic/Capric Triglyceride), so ist es vorteilhaft im Sinne der vorliegenden Erfindung, diese Substanz in einer Konzentration von 6 bis 25 Gewichts-%, bezogen auf das Gesamtgewicht der Zubereitung, einzusetzen.

Es ist erfindungsgemäß besonders bevorzugt, wenn die Zubereitung ausschließlich ölkomponenten mit einem Kontaktwinkel von größer/gleich 44° enthält.

Erfindungsgemäß Ausführungsformen der vorliegenden Erfindung sind dadurch gekennzeichnet, dass der Ethanolgehalt der Zubereitung von 20 bis 75 Gewichts-%, bezogen auf das Gesamtgewicht der Zubereitung, beträgt,

Erfindungsgemäß bevorzugte Ausführungsformen der vorliegenden Erfindung sind dadurch gekennzeichnet, dass der Ethanolgehalt der Zubereitung von 25 bis 70 Gewichts-%, bezogen auf das Gesamtgewicht der Zubereitung, beträgt.

Es ist vorteilhaft im Sinne der vorliegenden Erfindung, wenn die Gesamtkonzentration an den UV-Filtern 4-(tert.-Butyl)-4'-methoxydibenzoylmethan (INCI Butyl Methoxydibenzoylmethane), (2-[-4-(Diethylamino)-2-hydroxybenzoyl]Benzoesäurehexylester (INCI: Diethylamino Hydroxybenzoyl Hexyl Benzoate) und 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazine) in der Zubereitung von 0,5 bis 15 Gewichts-%, bezogen auf das Gesamtgewicht der Zubereitung, beträgt. Dabei ist eine Gesamtkonzentration von 1 bis 10 Gewichts-%, bezogen auf das Gesamtgewicht der Zubereitung, erfindungsgemäße bevorzugt.

Es ist erfindungsgemäß vorteilhaft, wenn das ethanolische Sonnenschutzmittel transparent ist. Dabei gilt eine Zubereitung erfindungsgemäß als transparent, wenn es möglich ist bei Tageslicht durch eine mit der erfindungsgemäßen Zubereitung gefüllten Einmal-Küvette (Firma Brand, 2,5ml, Wellenlängenbereich: 220nm-900nm) mit dem bloßen Auge zu schauen. Schriftzeichen (Schrifttyp Arial Schriftgröße 8), die sich unmittelbar hinter der Einmal-Küvette befinden, müssen klar erkennbar und lesbar sein.

Die erfindungsgemäße Zubereitung enthält kein 3-(4-Methylbenzyliden)-campher, 2-Hydroxy-4-methoxybenzophenon (INCI: Oxybenzon), Phenylen-1 ,4-bis-(2-benzimidazyl)-3,3'-5,5'-tetrasulfonsäuresalze; 1,4-di(2-oxo-10-Sulfo-3-bornyliden-methyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze, 3-Benzylidencampher; Terephthalidendicampher-sulfonsäure; 4-(Dimethylamino)-benzoesäure(2-ethylhexyl)ester; 4-Methoxybenzalmalon-säuredi(2-ethylhexyl)ester; 4-Methoxyzimtsäureisoamylester und 4-Methoxyzimtsäureethylhexylester also frei von diesen Inhaltsstoffen ist.

Andererseits sind erfindungsgemäß Ausführungsformen dadurch gekennzeichnet, dass die Zubereitung 2-Ethylhexyl 2-hydroxybenzoat (INCI: Ethylhexyl Salicylate) und/oder 3,3,5-Trimethylcyclohexyl 2-hydroxybenzoat (INCI: Homosalate) enthält.

Enthält die Zubereitung 2-Ethylhexyl 2-hydroxybenzoat (INCI: Ethylhexyl Salicylate), so ist es erfindungsgemäß von Vorteil, diese Verbindung in einer Konzentration von 1 bis 5 Gewichts-%, bezogen auf das Gesamtgewicht der Zubereitung, einzusetzen.

Enthält die Zubereitung 3,3,5-Trimethylcyclohexyl 2-hydroxybenzoat (INCI: Homosalate), so ist es erfindungsgemäß von Vorteil, diese Verbindung in einer Konzentration von 1 bis 5 Gewichts-%, bezogen auf das Gesamtgewicht der Zubereitung, einzusetzen.

Darüber hinaus kann es erfindungsgemäß von Vorteil sein, wenn die erfindungsgemäße Zubereitung Ethylhexyl-2-cyano-3,3-diphenylacrylat (INCI: Octocrylene) enthält.

Enthält die Zubereitung Ethylhexyl-2-cyano-3,3-diphenylacrylat (INCI: Octocrylen), so ist es erfindungsgemäß von Vorteil, diese Verbindung in einer Konzentration von 1 bis 10 Gewichts-%, bezogen auf das Gesamtgewicht der Zubereitung, einzusetzen.

Erfindungsgemäß vorteilhaft sind darüber hinaus Zubereitungen, die keine Alkandiole, Phenoxyethanol, Ethylhexylglycerin, Parabene, Methylisothiazolinon, Chlormethylisothiazolinon und DMDM-Hydantoin enthalten, also frei sind von diesen Inhaltsstoffen.

Es ist erfindungsgemäß vorteilhaft, wenn die erfindungsgemäße Zubereitung einen oder mehrere Parfümstoffe gewählt aus der Gruppe der Verbindungen Limonen, Citral, Linalool, alpha-Isomethylionon, Geraniol, Citronellol, 2-lsobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, Alpha-Methylionon, Amyl C Butylphenylmethylpropionalcinnamal, Amylsalicylat, Amylcinnamylalkohol, Anisalkohol, Benzoin, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citronellylmethylcrotonat, Citronenöl, Coumarin, Diethylsuccinat, Ethyllinalool, Eugenol, Evernia Furfuracea Extract, Evernia Prunastri Extract, Farnesol, Guajakholzöl, Hexylcinnamal, Hexylsalicylat, Hydroxycitronellal, Lavendelöl, Lemonenöl, Linaylacetat, Mandarinenöl, Menthyl PCA, Methylheptenon, Muskatnussöl, Rosmarinöl, süßes Orangenöl, Terpineol, Tonkabohnenöl, Triethylcitrat und/oder Vanillin enthält.

Außerdem ist es erfindungsgemäß von Vorteil, wenn die erfindungsgemäße Zubereitung Acrylates/Octylacrylamide Copolymer und/oder Vinylpyrrolidon/Hexadecen-Copolymer enthält.

Enthält die Zubereitung Acrylates/Octylacrylamide Copolymer, so ist es erfindungsgemäß von Vorteil, diese Verbindung in einer Konzentration von 0,5 bis 5 Gewichts-%, bezogen auf das Gesamtgewicht der Zubereitung, einzusetzen.

Es ist im Sinne der vorliegenden Erfindung darüber hinaus besonders vorteilhaft, wenn die erfindungsgemäße Zubereitung Glycerin enthält.

Ein Gehalt an Glycerin von mindestens 2,0 Gewicht-%, bezogen auf das Gesamtgewicht der Zubereitung, ist erfindungsgemäß besonders vorteilhaft.

Es ist erfindungsgemäß von besonderem Vorteil, wenn die erfindungsgemäße Zubereitung weniger als 3,0 Gewichts-% Wasser, bezogen auf das Gesamtgewicht der Zubereitung enthält.

Erfindungsgemäß vorteilhafte Ausführungsformen der vorliegenden Erfindung sind dadurch gekennzeichnet, dass die Zubereitung eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, Glycyrrhetinsäure, natürliche und/oder synthetische Isoflavonoide, Flavonoide, Kreatin, Kreatinin, Taurin, β-Alanin, Panthenol, Magnolol, Honokiol, Tocopherylacetat, Dihydroxyaceton; 8-Hexadecen-1,16-dicarbonsäure, Glycerylglycose, (2-Hydroxyethyl)harnstoff, Vitamin E bzw. Vitamin E Acetat, Hyaluronsäure und/oder deren Salze und/oder Licochalcon A enthält.

Die erfindungsgemäße Zubereitung kann erfindungsgemäß vorteilhaft als Gel, Pumpspray oder Aerosolspray vorliegen. Liegt die Zubereitung als Aerosolspray vor, so ist es erfindungsgemäß von besonderem Vorteil Propan, n-Butan, Isobutan oder deren Mischungen als Treibgas einzusetzen.

### Vergleichsversuch

Mit dem folgenden Versuch konnte der erfindungsgemäße Effekt beispielhaft belegt werden: Es wurden jeweils die speziellen Ölkomponenten zu einer UV-Filter enthaltenden Formulierung zugesetzt und die verfleckungsreduzierende Wirkung (Reduktion b*) im Vergleich zu einer Formulierung ohne erfindungsgemäße Ölkomponenten mittels beschriebener Methode bestimmt.

Es wurden verschiedene Sonnenschutzzubereitungen hinsichtlich der Bildung von gelben Flecken über einen in vitro Auftragungs-/ Waschzyklus untersucht. Es wurden dabei weiße vorgewaschene Baumwollmonitore (100% Baumwolle) verwendet. Dazu wurden je 50 mg der Test-Formulierung gleichmäßig auf PMMA Schönberg Platten (5,0 x 5,0 cm) verteilt und direkt mittels Andruck auf das Testtextil übertragen. Im Anschluss wurden die verfleckten Baumwollproben für 12h unter Laborbedingungen an der Luft getrocknet.

Nach der Trocknung erfolgte eine farbmetrische Charakterisierung der entstandenen Initial-Verfleckung durch Messung des Gelbgrades mit dem Farbmessgerät spectro-color (Dr. Lange); Farbmess-Software: spectral-QC, Version Messgeometrie: d/8°, Glanzkomponente ausgeschlossen, Lichtart: D65 (entsprechend mittlerem Tageslicht), Kalibrierstandard: LZM 268, Messöffnung: 10mm, Probenhintergrund: Unterlagepapier ohne optischen Aufheller, Prüfklima: 21°C (±1°C), 41% (±4%) rel. Luftfeuchte.

Zur Auswertung wurde die Veränderung des b-Wertes aus dem CIE-Lab Farbmesssystem herangezogen. Die b-Achse charakterisiert im CIE-Lab System den Farbeindruck Gelb/ Blau, wobei positive b-Werte für eine Zunahme des Gelbanteils stehen. Je höher der b-Wert desto größer ist der Gelbeindruck.

Nach dem Messvorgang erfolgte eine separate Wäsche der Testlappen im Färbe- und Waschechtheitsgerät Linitest Plus (Atlas) (60°C, 1h, 35rpm, Ariel Compact Pulverwaschmittel, 10 Metallkugeln als Beiladung, VES Wasser mit einer eingestellten Wasserhärte von 14°dH und einem Eisen-Ionengehalt von 0,1mg und einem Kupferionengehalt von 0,35mg/L) und im Anschluss ein Spülvorgang (20°C, 2x15min).

Nach Trocknung für 12h unter Laborbedingungen erfolgte erneut eine farbmetrische Charakterisierung der entstandenen Verfleckung durch Messung der Farbwerte wie bereits beschrieben mit dem Farbmessgerät spectro-color (Dr. Lange).

Die CIE-Lab System oder L*a*b*-Farbraum ist ein dreidimensionaler Messraum, in dem alle wahrnehmbaren Farben enthalten sind. Der Farbraum ist auf Grundlage der Gegenfarbentheorie konstruiert. Eine der wichtigsten Eigenschaften des L*a*b*-Farbmodells ist seine Geräteunabhängigkeit, das heißt, die Farben werden unabhängig von der Art ihrer Erzeugung und Wiedergabetechnik definiert.

Die entsprechende EU-Richtlinie ist DIN EN ISO 11664-4 "Farbmetrik - Teil 4: CIE 1976 L*a*b* Farbenraum", Die Koordinaten der CIELAB-Ebene werden gebildet aus dem Rot/ Grün-Wert a und dem Gelb/ Blau-Wert b. Die Helligkeitsachse L steht senkrecht auf dieser Ebene. Nach DIN 6174 sind L, a und b mit * zu schreiben, um sich gegen andere, z.B. das "Hunter-Lab"-System abzugrenzen.

| **INCI** | **Formel 1 nicht erfindungs -gemäß** | **Formel 2** | **Formel 3** | **Formel 4** | **Formel 5** |
|---|---|---|---|---|---|
| | **m [%]** | **m [%]** | **m [%]** | **m [%]** | **m [%]** |
| Butyl Methoxydibenzoylmethane | 4.50 | 4.50 | 4.50 | 4.50 | 4.50 |
| Ethylhexyl Salicylate | 4.50 | 4.50 | 4.50 | 4.50 | 4.50 |
| Octocrylene | 9.00 | 9.00 | 9.00 | 9.00 | 9.00 |
| Homosalate | 9.00 | 9.00 | 9.00 | 9.00 | 9.00 |
| Polysilicone-15 | 1.00 | 1.00 | 1.00 | 1.00 | 1.00 |
| Bis-Ethythexyloxyphenol Methoxyphenyl Triazine | 2,00 | 2.00 | 2.00 | 2.00 | 2.00 |
| Glycerin | 2.50 | 2.50 | 2.50 | 2.50 | 2.50 |
| Parfum | 0.70 | 0.70 | 0.70 | 0.70 | 0.70 |
| Acrylates/Octylecrylamide Copolymer | 1.00 | 1.00 | 1.00 | 1.00 | 1.00 |
| Dibutyl Adipate | 3.00 | | | | |
| Butylene Glycol Dicaprylate/Dicaprate | 3,00 | | | | |
| C12-15 Alkyl Benzoate | 9.50 | 15.50 | | | 4-00 |
| Octyldodecanol | | | 15.50 | | 5-80 |
| Caprylic/Capric Triglyceride | | | | 15.50 | 5.70 |
| Alcohol Denat | 50.30 | 50.30 | 50.30 | 50,30 | 50.30 |
| | | | | | |
| Reduktion b* [%] | | -10.00% | -6.10% | -5.00% | -5.30% |

Die Ergebnisse zeigen eine eindeutige Fleckenverminderung durch den Einsatz von Komplexbildnern im Vergleich zur Formulierung ohne die erfindungsgemäßen Ölkomponenten.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| INCI | Beispiel 1 | Beispiel 2 | Beispiel 3 | Beispiel 4 |
|---|---|---|---|---|
| | m [%] | m [%] | m [%] | m [%] |
| Butyl Methoxydibenzoylmethane | 4.5 | 4.5 | 2.5 | 2 |
| Ethylhexyl Salicylate | 4.5 | 4.5 | 2.5 | 2 |
| Octocrylene | 9 | 9.5 | 5 | 4 |
| Homosalate | 9 | 9.5 | 5 | 4 |
| Polysilicone-15 | 1 | | | |
| Bis-Ethylhexyloxyphenol Methoxyphenyl Triazine | 2 | 0.5 | | |
| Alcohol Denat. | 46.8 | 24.8 | 24.3 | 59.3 |
| Glycerin | 2.5 | | | 5 |
| Parfum | 0.7 | 0.7 | 0.7 | 0.7 |
| Acrylates/Octylacrylamide Copolymer + Aqua | 1 | 2 | 2 | 1 |
| Caprylic/Capric Triglyceride | 7 | 17 | 23 | 8 |
| Octyldodecanol | 7 | 17 | 23 | 8 |
| C12-15 Alkyl Benzoate | 5 | 10 | 12 | 6 |

## Patentansprüche

1. Ethanolisches, kosmetisches Sonnenschutzmittel enthaltend
a) einen oder mehrere UV-Filter gewählt aus der Gruppe der Verbindungen 4-(tert.-Butyl)-4'-methoxydibenzoylmethan (INCI Butyl Methoxydibenzoylmethane), (2-[-4-(Diethylamino)-2-hydroxybenzoyl] Benzoesäurehexylester (INCI: Diethylamino Hydroxybenzoyl Hexyl Benzoate) und 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin),
b) Ethanol in einem Gehalt von 20 bis 75 Gewichts-%, bezogen auf das Gesamtgewicht der Zubereitung,
c) ein oder mehrere Ölkomponenten mit einem Kontaktwinkel von größer/gleich 44°, wobei als Ölkomponenten mit einem Kontaktwinkel von größer/gleich 44° eine oder mehrere Verbindungen aus der Gruppe C12-15 Alkylbenzoate (INCI: C12-15 Alkyl Benzoate), 2-Octyldodecan-1-ol (INCI: Octyldodecanol), Capryl/Caprinsäure Triglyceride (INCI: Caprylic/Capric Triglyceride) gewählt werden und die Ölkomponenten mit einem Kontaktwinkel von größer/gleich 44° in einer Gesamtkonzentration von 15 bis 60 Gewichts-%, bezogen auf das Gesamtgewicht der Zubereitung,
d) 2-Ethylhexyl 2-hydroxybenzoat (INCI: Ethylhexyl Salicylate) und/oder 3,3,5-Trimethylcyclohexyl 2-hydroxybenzoat (INCI: Homosalate), wobei die Zubereitung kein 3-(4-Methylbenzyliden)-campher, 2-Hydroxy-4-methoxybenzophenon (INCI: Oxybenzon), Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze, 3-Benzylidencampher; Terephthalidendicampher-sulfonsäure; 4-(Dimethylamino)-benzoesäure(2-ethylhexyl)ester; 4-Methoxybenzalmalon-säuredi(2-ethylhexyl)ester; 4-Methoxyzimtsäureisoamylester und 4-Methoxyzimtsäureethylhexylester enthält, also frei von diesen Inhaltsstoffen ist.

2. Verwendung von Ölkomponenten mit einem Kontaktwinkel von größer/gleich 44° aus der Gruppe C12-15 Alkylbenzoate (INCI: C12-15 Alkyl Benzoate), 2-Octyldodecan-1-ol (INCI: Octyldodecanol), Capryl/Caprinsäure Triglyceride (INCI: Caprylic/Capric Triglyceride) in ethanolischen, kosmetischen Sonnenschutzmitteln enthaltend einen oder mehrere UV-Filter aus der Gruppe der Verbindungen 4-(tert.-Butyl)-4'-methoxy-dibenzoylmethan (INCI Butyl Methoxydibenzoylmethane), (2-[-4-(Diethylamino)-2-hydroxybenzoyl]-Benzoesäurehexylester (INCI: Diethylamino Hydroxybenzoyl Hexyl Benzoate) und 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin), zur Erleichterung der Auswaschbarkeit der UV-Lichtschutzfilter aus mit dem Sonnenschutzmittel kontaminierten Textilien.

3. Verwendung von Ölkomponenten mit einem Kontaktwinkel von größer/gleich 44° aus der Gruppe C12-15 Alkylbenzoate (INCI: C12-15 Alkyl Benzoate), 2-Octyldodecan-1-ol (INCI: Octyldodecanol), Capryl/Caprinsäure Triglyceride (INCI: Caprylic/Capric Triglyceride) in ethanolischen, kosmetischen Sonnenschutzmitteln enthaltend einen oder mehrere UV-Filter aus der Gruppe der Verbindungen 4-(tert.-Butyl)-4'-methoxy-dibenzoylmethan (INCI Butyl Methoxydibenzoylmethane), (2-[-4-(Diethylamino)-2-hydroxybenzoyl] Benzoesäurehexylester (INCI: Diethylamino Hydroxybenzoyl Hexyl Benzoate) und 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin), zur Reduzierung der durch das Sonnenschutzmittel ausgelösten Textilverfleckungen

4. Verfahren zur Erleichterung der Auswaschbarkeit von UV-Filter-Rückständen aus Textilien, die mit einem ethanolischen, kosmetischen Sonnenschutzmittel kontaminiert wurden, wobei dem ethanolischem Sonnenschutzmittel enthaltend einen oder mehrere UV-Filter aus der Gruppe der Verbindungen 4-(tert.-Butyl)-4'-methoxy-dibenzoylmethan (INCI Butyl Methoxydibenzoylmethane), (2-[-4-(Diethylamino)-2-hydroxybenzoyl]Benzoesäurehexylester (INCI: Diethylamino Hydroxybenzoyl Hexyl Benzoate) und 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin), ein oder mehrere Ölkomponenten mit einem Kontaktwinkel von größer/gleich 44° aus der Gruppe C12-15 Alkylbenzoate (INCI: C12-15 Alkyl Benzoate), 2-Octyldodecan-1-ol (INCI: Octyldodecanol), Capryl/Caprinsäure Triglyceride (INCI: Caprylic/Capric Triglyceride) zugesetzt wird.

5. Verfahren oder Verwendung nach einem der vorhergehenden Ansprüche, **dadurch gekennzeichnet, dass** der Ethanolgehalt der Zubereitung von 20 bis 75 Gewichts-%, bezogen auf das Gesamtgewicht der Zubereitung, beträgt.

6. Sonnenschutzmittel, Verfahren oder Verwendung nach einem der vorhergehenden Ansprüche, **dadurch gekennzeichnet, dass** die Gesamtkonzentration an den UV-Filtern 4-(tert.-Butyl)-4'-methoxydibenzoylmethan (INCI Butyl Methoxydibenzoylmethane), (2-[-4-(Diethylamino)-2-hydroxybenzoyl] Benzoesäurehexylester (INCI: Diethylamino Hydroxybenzoyl Hexyl Benzoate) und 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin) in der Zubereitung von 0,5 bis 15 Gewichts-%, bezogen auf das Gesamtgewicht der Zubereitung, beträgt.

7. Sonnenschutzmittel, Verfahren oder Verwendung nach einem der vorhergehenden Ansprüche, **dadurch gekennzeichnet dass** das ethanolische Sonnenschutzmittel transparent ist.

8. Verfahren oder Verwendung nach einem der vorhergehenden Ansprüche, **dadurch gekennzeichnet, dass** die Zubereitung kein 3-(4-Methylbenzyliden)-campher, 2-Hydroxy-4-methoxybenzophenon (INCI: Oxybenzon), Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze, 3-Benzylidencampher; Terephthalidendicampher-sulfonsäure; 4-(Dimethylamino)-benzoesäure(2-ethylhexyl)ester; 4-Methoxybenzalmalon-säuredi(2-ethylhexyl)ester; 4-Methoxyzimtsäureisoamylester und 4-Methoxyzimtsäureethylhexylester enthält, also frei von diesen Inhaltsstoffen ist.

9. Verfahren oder Verwendung nach einem der vorhergehenden Ansprüche, **dadurch gekennzeichnet, dass** die Zubereitung 2-Ethylhexyl 2-hydroxybenzoat (INCI: Ethylhexyl Salicylate) und/oder 3,3,5-Trimethylcyclohexyl 2-hydroxybenzoat (INCI: Homosalate) enthält.

10. Sonnenschutzmittel, Verfahren oder Verwendung nach einem der vorhergehenden Ansprüche, **dadurch gekennzeichnet, dass** die Zubereitung Ethylhexyl-2-cyano-3,3-diphenylacrylat (INCI: Octocrylen) enthält.

11. Sonnenschutzmittel, Verfahren oder Verwendung nach einem der vorhergehenden Ansprüche, **dadurch gekennzeichnet, dass** die Zubereitung keine Alkandiole, Phenoxyethanol, Ethylhexylglycerin, Parabene, Methylisothiazolinon, Chlormethylisothiazolinon und DMDM-Hydantoin enthält, also frei ist von diesen Inhaltsstoffen.

12. Sonnenschutzmittel, Verfahren oder Verwendung nach einem der vorhergehenden Ansprüche, **dadurch gekennzeichnet, dass** die Zubereitung einen oder mehrere Parfümstoffe gewählt aus der Gruppe der Verbindungen Limonen, Citral, Linalool, alpha-Isomethylionon, Geraniol, Citronellol, 2-Isobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, Alpha-Methylionon, Amyl C Butylphenylmethylpropionalcinnamal, Amylsalicylat, Amylcinnamylalkohol, Anisalkohol, Benzoin, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citronellylmethylcrotonat, Citronenöl, Coumarin, Diethylsuccinat, Ethyllinalool, Eugenol, Evernia Furfuracea Extract, Evernia Prunastri Extract, Farnesol, Guajakholzöl, Hexylcinnamal, Hexylsalicylat, Hydroxycitronellal, Lavendelöl, Lemonenöl, Linaylacetat, Mandarinenöl, Menthyl PCA, Methylheptenon, Muskatnussöl, Rosmarinöl, süßes Orangenöl, Terpineol, Tonkabohnenöl, Triethylcitrat und/oder Vanillin enthält.

13. Sonnenschutzmittel, Verfahren oder Verwendung nach einem der vorhergehenden Ansprüche, **dadurch gekennzeichnet, dass** die Zubereitung Acrylates/Octylacrylamide Copolymer enthält.

14. Sonnenschutzmittel, Verfahren oder Verwendung nach einem der vorhergehenden Ansprüche, **dadurch gekennzeichnet, dass** die Zubereitung weniger als 3,0 Gewichts-% Wasser, bezogen auf das Gesamtgewicht der Zubereitung enthält.

15. Sonnenschutzmittel, Verfahren oder Verwendung nach einem der vorhergehenden Ansprüche, **dadurch gekennzeichnet, dass** die Zubereitung ausschließlich Ölkomponenten mit einem Kontaktwinkel von größer/gleich 44° enthält.

## Claims

1. Ethanolic, cosmetic sunscreen comprising
a) one or more UV filters selected from the group of compounds comprising 4-tert-butyl-4'-methoxydibenzoylmethane (INCI: Butyl Methoxydibenzoylmethane), hexyl 2-[4-(diethylamino)-2-hydroxybenzoyl]benzoate (INCI: Diethylamino Hydroxybenzoyl Hexyl Benzoate) and 2,4-bis{[4-(2-ethylhexyloxy)-2-hydroxy]-phenyl-6-(4-methoxyphenyl)-1,3,5-triazine (INCI: Bis-Ethylhexyloxyphenol Methoxyphenyl Triazine),
b) ethanol in a content of 20% to 75% by weight based on the total weight of the preparation,
c) one or more oil components having a contact angle of greater than or equal to 44°, wherein the selected oil components having a contact angle of greater than or equal to 44° are one or more compounds from the group comprising C12-15 alkyl benzoates (INCI: C12-15 Alkyl Benzoate), 2-octyldodecan-1-ol (INCI: Octyldodecanol), caprylic/capric triglycerides (INCI: Caprylic/Capric Triglyceride) and the oil components having a contact angle of greater than or equal to 44° are in a total concentration of 15% to 60% by weight based on the total weight of the preparation,
d) 2-ethylhexyl 2-hydroxybenzoate (INCI: Ethylhexyl Salicylate) and/or 3,3,5-trimethylcyclohexyl 2-hydroxybenzoate (INCI: Homosalate), wherein the preparation contains no 3-(4-methylbenzylidene)camphor, 2-hydroxy-4-methoxybenzophenone (INCI: Oxybenzone), phenylene-1,4-bis(2-benzimidazyl)-3,3',5,5'-tetrasulfonic acid salts; 1,4-di(2-oxo-10-sulfo-3-bornylidenemethyl)benzene and salts thereof; 4-(2-oxo-3-bornylidenemethyl)benzenesulfonic acid salts; 2-methyl-5-(2-oxo-3-bornylidenemethyl)sulfonic acid salts, 3-benzylidenecamphor, terephthalidenedicamphorsulfonic acid; 2-ethylhexyl 4-(dimethylamino)benzoate; di(2-ethylhexyl) 4-methoxybenzalmalonate; isoamyl 4-methoxycinnamate, and ethylhexyl 4-methoxycinnamate, i.e. is free of these constituents.

2. Use of oil components having a contact angle of greater than or equal to 44° from the group comprising C12-15 alkyl benzoates (INCI: C12-15 Alkyl Benzoate), 2-octyldodecan-1-ol (INCI: Octyldodecanol), caprylic/capric triglycerides (INCI: Caprylic/Capric Triglyceride) in ethanolic cosmetic sunscreens comprising one or more UV filters from the group of compounds comprising 4-tert-butyl-4'-methoxydibenzoylmethane (INCI: Butyl Methoxydibenzoylmethane), hexyl 2-[4-(diethylamino)-2-hydroxybenzoyl]benzoate (INCI: Diethylamino Hydroxybenzoyl Hexyl Benzoate) and 2,4-bis{[4-(2-ethylhexyloxy)-2-hydroxy]-phenyl-6-(4-methoxyphenyl)-1,3,5-triazine (INCI: Bis-Ethylhexyloxyphenol Methoxyphenyl Triazine) for facilitating the washability of UV-protective filters out of textiles contaminated with the sunscreen.

3. Use of oil components having a contact angle of greater than or equal to 44° from the group comprising C12-15 alkyl benzoates (INCI: C12-15 Alkyl Benzoate), 2-octyldodecan-1-ol (INCI: Octyldodecanol), caprylic/capric triglycerides (INCI: Caprylic/Capric Triglyceride) in ethanolic cosmetic sunscreens comprising one or more UV filters from the group of compounds comprising 4-tert-butyl-4'-methoxydibenzoylmethane (INCI: Butyl Methoxydibenzoylmethane), hexyl 2-[4-(diethylamino)-2-hydroxybenzoyl]benzoate (INCI: Diethylamino Hydroxybenzoyl Hexyl Benzoate) and 2,4-bis{[4-(2-ethylhexyloxy)-2-hydroxy]-phenyl-6-(4-methoxyphenyl)-1,3,5-triazine (INCI: Bis-Ethylhexyloxyphenol Methoxyphenyl Triazine) for reducing textile spotting caused by the sunscreen.

4. Process for facilitating the washability of UV-filter residues out of textiles contaminated with an ethanolic cosmetic sunscreen, wherein one or more oil components having a contact angle of greater than or equal to 44° from the group comprising C12-15 alkyl benzoates (INCI: C12-15 Alkyl Benzoate), 2-octyldodecan-1-ol (INCI: Octyldodecanol), caprylic/capric triglycerides (INCI: Caprylic/Capric Triglyceride) is added to the ethanolic sunscreen comprising one or more UV filters from the group of compounds comprising 4-tert-butyl-4'-methoxydibenzoylmethane (INCI: Butyl Methoxydibenzoylmethane), hexyl 2-[4-(diethylamino)-2-hydroxybenzoyl]benzoate (INCI: Diethylamino Hydroxybenzoyl Hexyl Benzoate) and 2,4-bis{[4-(2-ethylhexyloxy)-2-hydroxy]-phenyl-6-(4-methoxyphenyl)-1,3,5-triazine (INCI: Bis-Ethylhexyloxyphenol Methoxyphenyl Triazine).

5. Process or use according to any of the preceding claims, **characterized in that** the ethanol content of the preparation is from 20% to 75% by weight based on the total weight of the preparation.

6. Sunscreen, process or use according to any of the preceding claims, **characterized in that** the total concentration in the preparation of the UV filters 4-tert-butyl-4'-methoxydibenzoylmethane (INCI: Butyl Methoxydibenzoylmethane), hexyl 2-[4-(diethylamino)-2-hydroxybenzoyl]benzoate (INCI: Diethylamino Hydroxybenzoyl Hexyl Benzoate) and 2,4-bis{[4-(2-ethylhexyloxy)-2-hydroxy]-phenyl-6-(4-methoxyphenyl)-1,3,5-triazine (INCI: Bis-Ethylhexyloxyphenol Methoxyphenyl Triazine) is from 0.5% to 15% by weight based on the total weight of the preparation.

7. Sunscreen, process or use according to any of the preceding claims, **characterized in that** the ethanolic sunscreen is transparent.

8. Process or use according to any of the preceding claims, **characterized in that** the preparation contains no 3-(4-methylbenzylidene)camphor, 2-hydroxy-4-methoxybenzophenone (INCI: Oxybenzone), phenylene-1,4-bis(2-benzimidazyl)-3,3',5,5'-tetrasulfonic acid salts; 1,4-di(2-oxo-10-sulfo-3-bornylidenemethyl)benzene and salts thereof; 4-(2-oxo-3-bornylidenemethyl)benzenesulfonic acid salts; 2-methyl-5-(2-oxo-3-bornylidenemethyl)sulfonic acid salts, 3-benzylidenecamphor, terephthalidenedicamphorsulfonic acid; 2-ethylhexyl 4-(dimethylamino)benzoate; di(2-ethylhexyl) 4-methoxybenzalmalonate; isoamyl 4-methoxycinnamate, and ethylhexyl 4-methoxycinnamate, i.e. is free of these constituents.

9. Process or use according to any of the preceding claims, **characterized in that** the preparation comprises 2-ethylhexyl 2-hydroxybenzoate (INCI: Ethylhexyl Salicylate) and/or 3,3,5-trimethylcyclohexyl 2-hydroxybenzoate (INCI: Homosalate).

10. Sunscreen, process or use according to any of the preceding claims, **characterized in that** the preparation comprises ethylhexyl 2-cyano-3,3-diphenylacrylate (INCI: Octocrylene).

11. Sunscreen, process or use according to any of the preceding claims, **characterized in that** the preparation contains no alkanediols, phenoxyethanol, ethylhexylglycerol, parabens, methylisothiazolinone, chloromethylisothiazolinone, and DMDM hydantoin, i.e. is free of these constituents.

12. Sunscreen, process or use according to any of the preceding claims, **characterized in that** the preparation contains one or more perfumes selected from the group of compounds comprising limonene, citral, linalool, alpha-isomethyl ionone, geraniol, citronellol, 2-isobutyl-4-hydroxy-4-methyltetrahydropyran, 2-tert-pentylcyclohexyl acetate, 3-methyl-5-phenyl-1-pentanol, 7-acetyl-1,1,3,4,4,6-hexamethyltetralin, adipic acid diesters, alpha-amylcinnamaldehyde, alpha-methyl ionone, amyl C butylphenyl methylpropionalcinnamal, amyl salicylate, amylcinnamyl alcohol, anise alcohol, benzoin, benzyl alcohol, benzyl benzoate, benzyl cinnamate, benzyl salicylate, bergamot oil, bitter orange oil, butylphenyl methylpropional, cardamom oil, cedrol, cinnamal, cinnamyl alcohol, citronellyl methylcrotonate, lemon oil, coumarin, diethyl succinate, ethyl linalool, eugenol, Evernia furfuracea extract, Evernia prunastri extract, farnesol, guaiac wood oil, hexylcinnamal, hexyl salicylate, hydroxycitronellal, lavender oil, lime oil, linalyl acetate, mandarin oil, menthyl PCA, methylheptenone, nutmeg oil, rosemary oil, sweet orange oil, terpineol, tonka bean oil, triethyl citrate, and/or vanillin.

13. Sunscreen, process or use according to any of the preceding claims, **characterized in that** the preparation comprises acrylates/octylacrylamide copolymer.

14. Sunscreen, process or use according to any of the preceding claims, **characterized in that** preparation contains less than 3.0% by weight of water based on the total weight of the preparation.

15. Sunscreen, process or use according to any of the preceding claims, **characterized in that** the preparation comprises only oil components having a contact angle of greater than or equal to 44°.

## Revendications

1. Agent de protection solaire cosmétique éthanolique contenant :
a) un ou plusieurs filtres UV choisis dans le groupe de composés constitué par le 4-(tert.-butyl)-4'-méthoxydibenzoylméthane (INCI : Butyl Methoxydibenzoylmethane), l'ester hexylique de l'acide (2-[-4-(diéthylamino)-2-hydroxybenzoyl]benzoïque (INCI : Diethylamino Hydroxybenzoyl Hexyl Benzoate) et la 2,4-bis-{[4-(2-éthyl-hexyloxy)-2-hydroxy]-phényl)-6-(4-méthoxyphényl)-1,3,5-triazine (INCI : Bis-Ethylhexyloxyphenol Methoxyphenyl Triazin),
b) de l'éthanol en une teneur de 20 à 75 % en poids, par rapport au poids total de la préparation,
c) un ou plusieurs composants huileux ayant un angle de contact supérieur ou égal à 44°, un ou plusieurs composés du groupe constitué par les benzoates d'alkyle en C12-15 (INCI : C12-15 Alkyl Benzoate), le 2-octyldodécan-1-ol (INCI : Octyldodecanol), les triglycériques de l'acide caprylique/caprique (INCI : Caprylic/Capric Triglycéride) étant choisis en tant que composants huileux ayant un angle de contact supérieur ou égal à 44° étant utilisés en une concentration totale de 15 à 60 % en poids, par rapport au poids total de la préparation,
d) du 2-hydroxybenzoate de 2-éthylhexyle (INCI : Ethylhexyl Salicylate) et/ou du 2-hydroxybenzoate de 3,3,5-triméthylcyclohexyle (INCI : Homosalate), la préparation ne contenant pas de 3-(4-méthylbenzylidène)-camphre, de 2-hydroxy-4-méthoxybenzophénone (INCI : Oxybenzon), de sels de l'acide phénylène-1,4-bis-(2-benzimidazyl)-3,3'-5,5'-tétrasulfonique ; de 1,4-di(2-oxo-10-sulfo-3-bornylidène-méthyl)-benzène et ses sels ; de sels de l'acide 4-(2-oxo-3-bornylidène-méthyl)benzène-sulfonique ; de sels de l'acide 2-méthyl-5-(2-oxo-3-bornylidène-méthyl)sulfonique, de 3-benzylidène-camphre ; d'acide téréphtalidène-dicamphre-sulfonique ; d'ester 2-éthylhexylique de l'acide 4-(diméthylamino)-benzoïque ; d'ester di(2-éthylhexylique) de l'acide 4-méthoxybenzalmalonique ; d'ester isoamylique de l'acide 4-méthoxycinnamique et d'ester éthylhexylique de l'acide 4-méthoxycinnamique, c'est-à-dire étant exempte de ces constituants.

2. Utilisation de composants huileux ayant un angle de contact supérieur ou égal à 44° du groupe constitué par les benzoates d'alkyle en C12-15 (INCI : C12-15 Alkyl Benzoate), le 2-octyldodécan-1-ol (INCI : Octyldodecanol), les triglycériques de l'acide caprylique/caprique (INCI : Caprylic/Capric Triglycéride) dans des agents de protection solaire cosmétiques éthanoliques contenant un ou plusieurs filtres UV du groupe de composés constitué par le 4-(tert.-butyl)-4'-méthoxydibenzoylméthane (INCI : Butyl Methoxydibenzoylmethane), l'ester hexylique de l'acide (2-[-4-(diéthylamino)-2-hydroxybenzoyl]benzoïque (INCI : Diethylamino Hydroxybenzoyl Hexyl Benzoate) et la 2,4-bis-{[4-(2-éthyl-hexyloxy)-2-hydroxy]-phényl)-6-(4-méthoxyphényl)-1,3,5-triazine (INCI : Bis-Ethylhexyloxyphenol Methoxyphenyl Triazin), pour faciliter la rinçabilité des filtres de protection contre la lumière UV à partir de textiles contaminés avec l'agent de protection solaire.

3. Utilisation de composants huileux ayant un angle de contact supérieur ou égal à 44° du groupe constitué par les benzoates d'alkyle en C12-15 (INCI : C12-15 Alkyl Benzoate), le 2-octyldodécan-1-ol (INCI : Octyldodecanol), les triglycériques de l'acide caprylique/caprique (INCI : Caprylic/Capric Triglycéride) dans des agents de protection solaire cosmétiques éthanoliques contenant un ou plusieurs filtres UV du groupe de composés constitué par le 4-(tert.-butyl)-4'-méthoxydibenzoylméthane (INCI : Butyl Methoxydibenzoylmethane), l'ester hexylique de l'acide (2-[-4-(diéthylamino)-2-hydroxybenzoyl]benzoïque (INCI : Diethylamino Hydroxybenzoyl Hexyl Benzoate) et la 2,4-bis-{[4-(2-éthyl-hexyloxy)-2-hydroxy]-phényl)-6-(4-méthoxyphényl)-1,3,5-triazine (INCI : Bis-Ethylhexyloxyphenol Methoxyphenyl Triazin), pour réduire les taches de textiles causées par l'agent de protection solaire.

4. Procédé pour faciliter la rinçabilité de résidus de filtres UV à partir de textiles qui ont été contaminés avec un agent de protection solaire cosmétique éthanolique, selon lequel un ou plusieurs composants huileux ayant un angle de contact supérieur ou égal à 44° du groupe constitué par les benzoates d'alkyle en C12-15 (INCI : C12-15 Alkyl Benzoate), le 2-octyldodécan-1-ol (INCI : Octyldodecanol), les triglycériques de l'acide caprylique/caprique (INCI : Caprylic/Capric Triglycéride) sont ajoutés à l'agent de protection solaire éthanolique contenant un ou plusieurs filtres UV du groupe de composés constitué par le 4-(tert.-butyl)-4'-méthoxydibenzoylméthane (INCI : Butyl Methoxydibenzoylmethane), l'ester hexylique de l'acide (2-[-4-(diéthylamino)-2-hydroxybenzoyl]benzoïque (INCI : Diethylamino Hydroxybenzoyl Hexyl Benzoate) et la 2,4-bis-{[4-(2-éthyl-hexyloxy)-2-hydroxy]-phényl)-6-(4-méthoxyphényl)-1,3,5-triazine (INCI : Bis-Ethylhexyloxyphenol Methoxyphenyl Triazin).

5. Procédé ou utilisation selon l'une quelconque des revendications précédentes, caractérisé ou **caractérisée en ce que** la teneur en éthanol de la préparation est de 20 à 75 % en poids, par rapport au poids total de la préparation.

6. Agent de protection solaire, procédé ou utilisation selon l'une quelconque des revendications précédentes, caractérisé ou **caractérisée en ce que** la concentration totale en les filtres UV 4-(tert.-butyl)-4'-méthoxydibenzoylméthane (INCI : Butyl Methoxydibenzoylmethane), ester hexylique de l'acide (2-[-4-(diéthylamino)-2-hydroxybenzoyl]benzoïque (INCI : Diethylamino Hydroxybenzoyl Hexyl Benzoate) et 2,4-bis-{[4-(2-éthyl-hexyloxy)-2-hydroxy]-phényl)-6-(4-méthoxyphényl)-1,3,5-triazine (INCI : Bis-Ethylhexyloxyphenol Methoxyphenyl Triazin) dans la préparation est de 0,5 à 15 % en poids, par rapport au poids total de la préparation.

7. Agent de protection solaire, procédé ou utilisation selon l'une quelconque des revendications précédentes, caractérisé ou **caractérisée en ce que** l'agent de protection solaire éthanolique est transparent.

8. Procédé ou utilisation selon l'une quelconque des revendications précédentes, caractérisé ou **caractérisée en ce que** la préparation ne contient pas de 3-(4-méthylbenzylidène)-camphre, de 2-hydroxy-4-méthoxybenzophénone (INCI : Oxybenzon), de sels de l'acide phénylène-1,4-bis-(2-benzimidazyl)-3,3'-5,5'-tétrasulfonique ; de 1,4-di(2-oxo-10-sulfo-3-bornylidène-méthyl)-benzène et ses sels ; de sels de l'acide 4-(2-oxo-3-bornylidène-méthyl)benzène-sulfonique ; de sels de l'acide 2-méthyl-5-(2-oxo-3-bornylidène-méthyl)sulfonique, de 3-benzylidène-camphre ; d'acide téréphtalidène-dicamphre-sulfonique ; d'ester 2-éthylhexylique de l'acide 4-(diméthylamino)-benzoïque ; d'ester di(2-éthylhexylique) de l'acide 4-méthoxybenzalmalonique ; d'ester isoamylique de l'acide 4-méthoxycinnamique et d'ester éthylhexylique de l'acide 4-méthoxycinnamique, c'est-à-dire est exempte de ces constituants.

9. Procédé ou utilisation selon l'une quelconque des revendications précédentes, caractérisé ou **caractérisée en ce que** la préparation contient du 2-hydroxybenzoate de 2-éthylhexyle (INCI : Ethylhexyl Salicylate) et/ou du 2-hydroxybenzoate de 3,3,5-triméthylcyclohexyle (INCI : Homosalate).

10. Agent de protection solaire, procédé ou utilisation selon l'une quelconque des revendications précédentes, caractérisé ou **caractérisée en ce que** la préparation contient de l'acrylate d'éthylhexyl-2-cyano-3,3-diphényle (INCI : Octocrylen).

11. Agent de protection solaire, procédé ou utilisation selon l'une quelconque des revendications précédentes, caractérisé ou **caractérisée en ce que** la préparation ne contient pas d'alcane-diols, de phénoxyéthanol, d'éthylhexylglycérine, de parabènes, de méthylisothiazolinone, de chlorométhylisothiazolinone et de DMDM-hydantoïne, c'est-à-dire est exempte de ces constituants.

12. Agent de protection solaire, procédé ou utilisation selon l'une quelconque des revendications précédentes, caractérisé ou **caractérisée en ce que** la préparation contient un ou plusieurs parfums choisis dans le groupe de composés constitué par le limonène, le citral, le linalool, l'alpha-isométhylionone, le géraniol, le citronellol, le 2-isobutyl-4-hydroxy-4-méthyltétrahydropyrane, l'acétate de 2-tert-pentylcyclohexyle, le 3-méthyl-5-phényl-1-pentanol, la 7-acétyl-1,1,3,4,4,6-hexaméthyltétraline, les diesters de l'acide adipique, l'alpha-amylcinnamaldéhyde, l'alpha-méthylionone, l'amyl C butylphénylméthylpropionalcinnamal, le salicylate d'amyle, l'alcool amylcinnamylique, l'alcool anisique, la benzoïne, l'alcool benzylique, le benzoate de benzyle, le cinnamate de benzyle, le salicylate de benzyle, l'huile de bergamote, l'huile d'orange amère, le butylphénylméthylpropional, l'huile de cardamome, le cédrol, le cinnamal, l'alcool cinnamylique, le crotonate de citronellylméthyle, l'huile de citron, la coumarine, le succinate de diéthyle, l'éthyllinalool, l'eugénol, l'extrait d'Evernia Furfuracea, l'extrait d'Evernia Prunastri, le farnésol, l'huile de bois de gaïac, l'hexylcinnamal, le salicylate d'hexyle, l'hydroxycitronellal, l'huile de lavande, l'huile de citron vert, l'acétate de linalyle, l'huile de mandarine, le menthyl PCA, la méthylhepténone, l'huile de noix de muscade, l'huile de romarin, l'huile d'orange douce, le terpinéol, l'huile de fève de Tonka, le citrate de triéthyle et/ou la vanilline.

13. Agent de protection solaire, procédé ou utilisation selon l'une quelconque des revendications précédentes, caractérisé ou **caractérisée en ce que** la préparation contient du copolymère acrylates/octylacrylamide.

14. Agent de protection solaire, procédé ou utilisation selon l'une quelconque des revendications précédentes, caractérisé ou **caractérisée en ce que** la préparation contient moins de 3,0 % en poids d'eau, par rapport au poids total de la préparation.

15. Agent de protection solaire, procédé ou utilisation selon l'une quelconque des revendications précédentes, caractérisé ou **caractérisée en ce que** la préparation contient exclusivement des composants huileux ayant un angle de contact supérieur ou égal à 44°.
